# EUROPEAN PATENT SPECIFICATION

(11) **EP 2 177 554 B1**
(45) Date of publication and mention of the grant of the patent: **16.04.2014**
(21) Application number: 09252409.9
(22) Date of filing: 14.10.2009
(51) Int. Cl.: C08G 63/06

(54) **Hydroxamate-initiated polymers**
Mit Hydroxamat initiierte Polymere
Polymères initiés par hydroxamate

(30) Priority: 15.10.2008 US 105479 P; 02.10.2009 US 572362
(43) Date of publication of application: 21.04.2010
(73) Proprietor: Covidien LP, Mansfield, MA 02048 (US)
(72) Inventor: Stopek, Joshua, Yalesville, CT 06492 (US); Hadba, Ahmad, Middlefield, CT 06455 (US)
(74) Representative: Soames, Candida Jane

(56) References cited:
- WO-A1-2005/000915
- WO-A1-2006/002506
- JP-A- 9 221 420

## Description

### TECHNICAL FIELD

The present disclosure relates to polymers initiated with hydroxamates, compositions containing such polymers, and articles made from or coated with such polymers or compositions.

### BACKGROUND OF RELATED ART

Matrix metalloproteinases (MMPs) are neutral zinc-dependent endopeptidases with substrate specificity for most extracellular matrix molecules, including collagens, gelatins, fibronectin, laminin and proteoglycan. They depend upon zinc for their catalytic activity.

Most cells do not express MMPs in vivo. Instead, growth factors, hormones, inflammatory cytokines, cell-matrix interactions and cellular transformation regulate their expression. Although the secretory granules of neutrophils and eosinophils are known to store some MMPs, most cell types normally synthesize very low quantities of MMPs.

MMPs share some common structural characteristics that include a signal sequence, an amino-terminal pro-peptide domain, a catalytic zinc binding domain, a proline-rich hinge region, and a carboxy-terminal hemopexin-like domain.

Extracellular matrix degradation is a normal event in the physiological remodeling associated with morphogenesis, reproduction, and in growth and maintenance processes such as cell migration, angiogenesis, and tissue regeneration. During inflammation and in several disease situations, however, excess MMPs may degrade the surrounding proteinaceous matrix, which may result in the destruction or weakening of connective tissue, unregulated cell migration/invasion, and/or tissue fibrosis. For example, connective tissue weakening or destruction may result in diseases such as rheumatoid arthritis, osteoarthritis, chronic periodontis, and arterial and cardiac aneurysm. Accordingly, MMP inhibitors have been used to treat osteoporosis, osteoarthritis, human chronic periodontal disease and various types of aneurysms.

Medical devices and compositions that can reduce inflammation and prevent the degradation of the extracellular matrix by MMPs, particularly in response to a disease or injury, remain desirable.

JP 09 221 420 discloses medical preparations including a polyhydroxyalkanoate and a hydroxamic acid derivative.

### SUMMARY

The present disclosure provides processes for producing polymers with hydroxamates, and polymers produced thereby. Medical devices formed of such polymers are also provided.

In embodiments a hydroxamate-initiated polymer is provided, wherein:
(i) the hydroxamate has the formula: 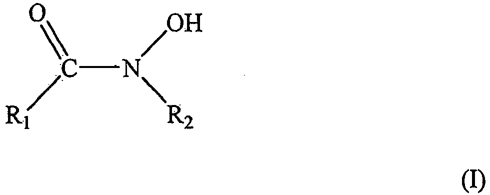 wherein R₁ is selected from the group consisting of vinyl groups, hydroxy acrylate groups, hydroxy methacrylate groups, acrylamide, methacrylamide, alkyl amines, alkyl groups, alkoxy groups, alkenyl groups, polymers terminated with the foregoing groups, and combinations thereof, and R₂ comprises hydrogen, and
(ii) the hydroxamate-initiated polymer comprises a polymeric component, said polymeric component:
   a) being formed from one or more lactones selected from the group consisting of lactide, glycolide, trimethylene carbonate, tetramethylene carbonate, dimethyl trimethylene carbonate, dioxanones, dioxepanones, caprolactone, valerolactone, and combinations thereof, optionally together with one or more monomer selected from absorbable cyclic amides, absorbable cyclic ether-esters derived from crown ethers, hydroxyl acids capable of esterification, polyalkyl ethers, and combinations thereof, or
   b) being selected from polyesters, polyamides, polyester/polyethers, homopolymers thereof, copolymers thereof, and combinations thereof; and
wherein said hydroxamate is attached via a hydrolytically degradable bond at the head of the polymeric component.

A method of the present disclosure comprises contacting one or more monomers with a hydroxamate-containing initiator under polymerizing conditions, the hydroxamate-containing initiator having the formula: 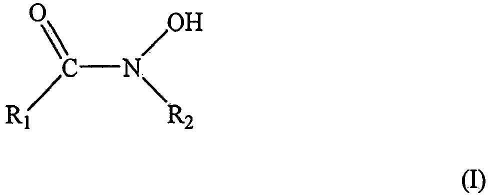 wherein R₁ is selected from the group consisting of vinyl groups, hydroxy acrylate groups, hydroxy methacrylate groups, acrylamide, methacrylamide, alkyl amines, alkyl groups, alkoxy groups, alkenyl groups, polymers terminated with the foregoing groups, and combinations thereof, and R₂ comprises hydrogen; said one or more monomers being:
a) one or more lactone monomers selected from the group consisting of lactide, glycolide, trimethylene carbonate, tetramethylene carbonate, dimethyl trimethylene carbonate, dioxanones, dioxepanones, caprolactone, valerolactone, and combinations thereof, optionally together with one or more monomer selected from absorbable cyclic amides, absorbable cyclic ether-esters derived from crown ethers, hydroxyl acids capable of esterification, polyalkyl ethers, and combinations thereof, or
b) one or more monomers that form polyesters, polyamides, polyester/ polyethers, homopolymers thereof, copolymers thereof, and combinations thereof; and
   recovering a polymer comprising a polymeric component and the hydroxamate attached via a hydrolytically degradable bond at the head of the polymeric component.

Medical devices of the present disclosure comprise a hydroxamate-initiated polymer wherein:
(i) the hydroxamate initiator has the formula: 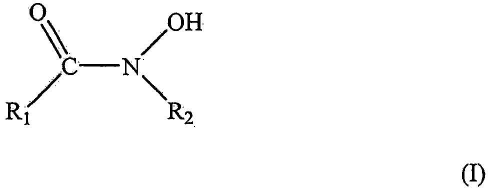 wherein R₁ is selected from the group consisting of vinyl groups, hydroxy acrylate groups, hydroxy methacrylate groups, acrylamide, methacrylamide, alkyl amines, alkyl groups, alkoxy groups, alkenyl groups, polymers terminated with the foregoing groups, and combinations thereof, and R₂ comprises hydrogen; and
(ii) the hydroxamate-initiated polymer comprises a polymeric component, said polymeric component:
   a) being formed from one or more lactones selected from the group consisting of lactide, glycolide, trimethylene carbonate, tetramethylene carbonate, dimethyl trimethylene carbonate, dioxanones, dioxepanones, caprolactone, valerolactone, and combinations thereof, optionally together with one or more monomer selected from absorbable cyclic amides, absorbable cyclic ether-esters derived from crown ethers, hydroxyl acids capable of esterification, polyalkyl ethers, and combinations thereof, or
   b) being selected from polyesters, polyamides, polyester/polyethers, hompolymers thereof, copolymers thereof, and combinations thereof; and
wherein said hydroxamate is attached via a hydrolytically degradable bond at the head of the polymeric component.

Medical devices formed of the polymers of the present disclosure may include sutures, surgical meshes, contact lenses, intraocular lenses, staples, clips, buttresses, lapbands, catheters, bandages, stents, grafts, stent/grafts, knotless wound closures, sealants, adhesives, anti-adhesion devices, anchors, tunnels, bone fillers, synthetic tendons, synthetic ligaments, grafts, tissue scaffolds, pins, screws, orthopedic hardware, pacers, pacemakers, and implants.

In other embodiments, substrates, including medical devices, may possess a coating on at least a portion of a surface of the medical device, wherein the coating includes a hydroxamate-initiated polymer.

### DETAILED DESCRIPTION

The present disclosure provides polymers initiated with hydroxamates and compositions containing such polymers.

The present hydroxamate-initiated polymers may be bioabsorbable or nonabsorbable. In embodiments, a bioabsorbable polymer may be utilized in a composition of the present disclosure. As used herein the term "polymer" may be used interchangeably with "polymeric component" and includes homopolymers and copolymers including, but not limited to, random, block or segmented copolymers.

In embodiments, polymers may be formed by the ring opening polymerization of one or more cyclic lactones using a hydroxamate as an initiator. In such embodiments, one or more lactone monomers may be utilized to form the hydroxamate-initiated polymers of the present disclosure. Suitable lactone monomers are lactide; glycolide; trimethylene carbonate; tetramethylene carbonate; dimethyl trimethylene carbonate; dioxanone; dioxepanone; caprolactone; valerolactone; and combinations thereof.

In embodiments, additional monomers may be added to the lactone monomers thereby forming a copolymer. Monomers which can be copolymerized with the lactones described above are absorbable cyclic amides; absorbable cyclic ether-esters derived from crown ethers; hydroxyacids capable of esterification including alpha hydroxy acids (such as glycolic acid and lactic acid) and beta hydroxyacids (such as beta hydroxybutyric acid and gamma hydroxyvaleric acid); polyalkyl ethers (such as polyethylene glycol); and combinations thereof.

In embodiments, monomers that form non-absorbable hydroxamate-initiated polymers can be used. Such non-absorbable hydroxamate-initiated polymers are polyesters, polyamides, polyester/polyethers, homopolymers thereof, copolymers thereof, and combinations thereof.

The polymers of the present disclosure are formed by polymerizing one or more monomers in the presence of a hydroxamate initiator. Hydroxamates for use in initiating the formation of polymers in accordance with the present disclosure include, for example, polymers or compositions possessing a hydroxamate group of the following formula (I): 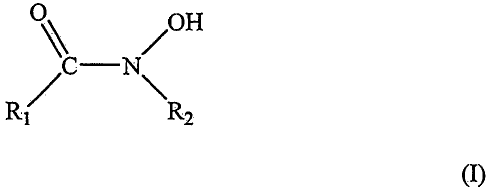 wherein R₁ is selected from the group consisting of vinyl groups including vinyl acetate; hydroxy alkyl acrylates; hydroxy methacrylate groups including hydroxy alkyl methacrylates, hydroxyethyl methacrylates, acrylamides, methacrylamides, alkyl amines, alkyl groups; alkoxy groups; alkenyl groups; polymers terminated with any of the above groups, such as vinyl alcohols, hydroxyethyl methacrylates, and combinations thereof; and R₂ comprises hydrogen.

As used herein, "alkyl", used either alone or in compound words such as "haloalkyl" or "alkylthio", includes straight chain or branched C₁₋₁₂ alkyl groups. Examples include methyl, ethyl, propyl, isopropyl and the like.

As used herein, "alkoxy" includes straight chain or branched alkoxy, in embodiments C₁₋₁₂ alkoxy such as methoxy, ethoxy, n-propoxy, isopropoxy and butoxy isomers.

As used herein, "alkenyl" includes groups formed from straight chain, branched or mono- or polycyclic alkenes including ethylenically mono- or poly-unsaturated alkyl or cycloalkyl groups as previously defined, in embodiments C₂₋₁₂ alkenyl. Examples of alkenyl include vinyl; allyl; 1-methylvinyl; butenyl; iso-butenyl; 3-methyl-2-butenyl; 1-pentenyl; cyclopentenyl; 1-methyl-cyclopentenyl; 1-hexenyl; 3-hexenyl; cyclohexenyl; 1-heptenyl; 3-heptenyl;1-octenyl; cyclooctenyl; 1-nonenyl; 2-nonenyl; 3-nonenyl; 1-decenyl; 3-decenyl; 1,3-butadienyl; 1-4,pentadienyl; 1,3-cyclopentadienyl; 1,3-hexadienyl; 1,4-hexadienyl; 1,3-cyclohexadienyl; 1,4-cyclohexadienyl; 1,3-cycloheptadienyl; 1,3,5-cycloheptatrienyl; or 1,3,5,7-cyclooctatetraenyl.

Methods for forming such hydroxamate functional compositions are within the purview of those skilled in the art. For example, in embodiments, a hydroxamate functional polymer may be produced by the surface modification of cross-linked polymethacrylic acid (PMAA)-co-methyl methacrylate (MAA) beads, thus producing a hydroxamate functional polymer, i.e., PMAA-MMA-hydroxamate.

In embodiments, polymerizable hydroxamate monomers may be synthesized which, in turn, may be utilized as the hydroxamate initiator. In some embodiments, referring to formula I above, R₁ may be CH₂=C-CH₃ and R₂ comprises hydrogen. In other embodiments, the hydroxamate monomer may be utilized to synthesize a hydroxamate homopolymer, or may be copolymerized with any other suitable comonomers to produce copolymers suitable for use as a hydroxamate initiator.

Hydroxamate homopolymers synthesized from the above hydroxamate monomer can also be grafted onto any derivatizable polymer. The resulting hydroxamate functional composition, whether a monomer, homopolymer, or copolymer, corresponding to formula I above, may then be used as a hydroxamate initiator to produce hydroxamate functional polymers.

It should, of course, be understood that two or more hydroxamates may be used as the initiator.

Conditions for conducting polymerization are within the purview of those skilled in the art. Where the monomers employed are cyclic lactones, for example, monomers can be dried, mixed in a reaction vessel with a hydroxamate initiator and a suitable polymerization catalyst, if desired, and heated at temperatures from about 160° C to about 200° C for a period of time from about 4 hours to about 30 hours.

In embodiments, a hydroxamate of formula I above may be reacted with a ring compound, and thus undergo ring opening polymerization (ROP). For example, a compound of Formula I may be reacted with a compound of the following formula: 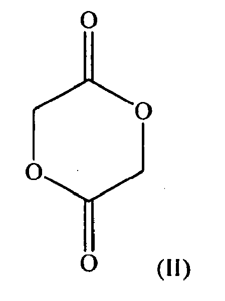 to form a hydroxamate of the following formula: 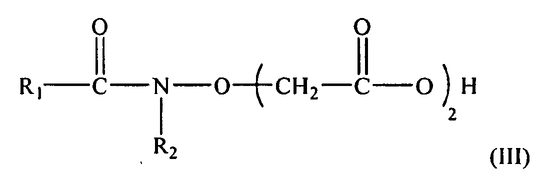 where R₁ and R₂ are as defined above for formula I.

The resulting hydroxamate functional polymers may have MMP-inhibiting properties.

For example, in embodiments, compositions possessing the hydroxamate functional polymers may be used in wound treatment, or in the formation of medical devices and implants. Chronic wounds may take months or years to heal due, in part, to high levels of MMPs that degrade the newly formed matrix even as it is synthesized. The hydroxamate functional polymers of the present disclosure, due to the presence of the hydroxamate group, may inhibit the activity of the MMPs in or adjacent a wound, thereby promoting healing.

Similarly, angiogenesis or vasculogenesis of tumors and the formation of metastases require cell migration and invasion, which are enabled by the release of pro-MMPs. The hydroxamate functional polymers of the present disclosure, which counteract those MMPs, may thus be suitable for minimizing angiogenesis and/or vascularization of tumors.

Furthermore tissue remodeling occurs secondary to secretion or expression of MMPs. Thus, blood vessels associated with wound repair are resorbed or ischemic tissue is destroyed by MMP action. The hydroxamate functional polymers of the present disclosure, which counteract those MMPs, may thus be suitable to enhance wound repair.

The activity of MMPs is also essential for many of the processes involved in atherosclerotic plaque formation (infiltration of inflammatory cells, angiogenesis, and smooth muscle cell migration and proliferation). Elevated levels of MMPs are expressed in human atherosclerotic plaque and at the sites of aneurysm. Furthermore, matrix degradation by MMPs may cause the plaque instability and rupture that leads to the clinical symptoms of atherosclerosis. The, hydroxamate functional polymers of the present disclosure, which counteract those MMPs, may thus be suitable to reduce the formation of atherosclerotic plaques and the incidence of rupture at the sites of aneurysm.

In the context of arthritis, a similar role for activated MMPs in cartilage degradation has been demonstrated. Elevated concentrations and activities of several MMPs including MMP-1, MMP-3, MMP-8 and MMP-13, as well as aggrecanase (another metalloproteinase) have been identified in the synovial fluid of osteoarthritis and rheumatoid arthritis patients. The hydroxamate functional polymers of the present disclosure, which counteract those MMPs, may thus be suitable to treat arthritis and/or minimize the degradation of cartilage.

There is also accumulating evidence that an increase in the proportion of active MMPs is associated with the progression of restenosis following vascular interventions such as balloon angioplasty or intra-coronary stenting, for the treatment of coronary artery disease. In contrast to the non-diseased vessel wall, which constitutively expresses only pro-(inactive) MMP-2, injured or atherosclerotic arteries demonstrate a dramatic increase in MMP-2 activity. This occurs in conjunction with induced expression of MMPs-3, -7, -9, -12, and -13. The hydroxamate functional polymers of the present disclosure, which counteract those MMPs, may thus be utilized to reduce restenosis.

The hydroxamate functional polymers of the present disclosure may inactivate MMPs by binding the zinc at the active center of the enzymes. With multiple point attachments, the hydroxamates behave like a molecular magnet for zinc.

In embodiments, the hydroxamate functional polymers of the present disclosure may bind to the active form of MMPs, without any specificity for particular MMP types. In other embodiments, the hydroxamate functional polymer may provide preferential binding to active forms of MMPs in the local tissue environment. This may be advantageous because it specifically targets one stage in the MMP regulatory cascade, namely that directly preceding matrix degradation. In addition, selective binding reduces the risk of over inhibition which would delay healing by preventing a healthy rate of tissue turnover and essential processes such as cell migration and angiogenesis.

The resulting hydroxamate functional polymers may be suitable for coating other materials, made into a solid material after conventional thermoplastic processing (molding, extrusion, etc.), or made into beads or nanoparticles by spray drying, solvent evaporation or any other conventional polymer processing method.

For example, small beads of hydroxamate functional polymer may be injected in the vicinity of diseased or damaged tissue. Alternatively, hydroxamate functional polymers can be incorporated into devices in contact with tissue, for example, compounding within a polymer resin or as a device coating.

The hydroxamate-initiated polymers thus produced have a hydroxamate attached via a hydrolytically degradable bond at the head of the polymer chain. Advantageously, upon hydrolysis, the present hydroxamate-initiated polymers release low concentrations of the hydroxamate, thus providing inhibition of MMP activity at the site of implantation or injury to which the hydroxamate functional polymer or an article including the hydroxamate functional polymer is applied.

The present hydroxamate-initiated polymers can be formed into articles using any known technique, such as, for example, extrusion, molding and/or solvent casting. Methods for forming articles with the hydroxamate-initiated polymer of the present disclosure are within the purview of those skilled in the art. The polymers can be used alone, blended with other polymers, either absorbable or non-absorbable.

In embodiments, surgical articles, also referred to herein as medical devices, can be manufactured from the hydroxamate-initiated polymers described herein. Suitable medical devices include, but are not limited to, clips and other fasteners, staples, sutures, pins, screws, prosthetic devices, wound dressings, bandages, drug delivery devices, anastomosis rings, surgical blades, contact lenses, intraocular lenses, surgical meshes, stents, stent coatings, grafts, catheters, stent/grafts, knotless wound closures, sealants, adhesives, anti-adhesion devices, anchors, tunnels, bone fillers, synthetic tendons, synthetic ligaments, tissue scaffolds, stapling devices, buttresses, laparoscopic bands (lap bands), orthopedic hardware, pacers, pacemakers, drug delivery devices, soft tissue repair devices including mesh fixation, and other implants and implantable devices.

Fibers can also be made from the present hydroxamate-initiated polymers. In embodiments, fibers made of hydroxamate-initiated polymers of the present disclosure may be knitted or woven with other fibers, either absorbable or non-absorbable fibers, to form textiles. The fibers also can be made into non-woven materials to form fabrics, such as meshes and felts.

In embodiments, combinations of the hydroxamate-initiated polymers, optionally in combination with additional hydroxamates encompassed by formula (I) above, may also be utilized in a coating by, in embodiments, admixing free hydroxamates with the hydroxamate-initiated polymer of the present disclosure.

In some embodiments, compositions may be formed by combining the hydroxamate-initiated polymers with other components. In embodiments, compositions including the hydroxamate-initiated polymers can be used as a coating for surgical devices. Coatings of surgical and/or medical devices may, in embodiments, be on at least a portion of a surface of the medical device. Such surfaces may include exterior surfaces, interior surfaces, and combinations thereof.

In embodiments, coating compositions may contain the present hydroxamate-initiated polymers combined with a fatty acid component, such as a fatty acid, a fatty acid salt, or a salt of a fatty acid ester. Suitable fatty acids may be saturated or unsaturated, and include higher fatty acids having more than about 12 carbon atoms. Suitable saturated fatty acids include, for example, stearic acid, palmitic acid, myristic acid and lauric acid. Suitable unsaturated fatty acids include oleic acid, linoleic acid, and linolenic acid. In addition, an ester of fatty acids, such as sorbitan tristearate or hydrogenated castor oil, may be used.

Suitable fatty acid salts include the polyvalent metal ion salts of C₆ and higher fatty acids, in embodiments those having from about 12 to about 22 carbon atoms, and mixtures thereof. Fatty acid salts including the calcium, magnesium, barium, aluminum, and zinc salts of stearic, palmitic and oleic acids may be useful in some embodiments of the present disclosure. Some useful salts include commercial "food grade" calcium stearate which contains a mixture of about one-third C₁₆ and two-thirds C₁₈ fatty acids, with small amounts of the C₁₄ and C₂₂ fatty acids.

Suitable salts of fatty acid esters which may be included in the compositions of the present disclosure include calcium, magnesium, aluminum, barium, or zinc stearoyl lactylate; calcium, magnesium, aluminum, barium, or zinc palmityl lactylate; and/or calcium, magnesium, aluminum, barium, or zinc olelyl lactylate. In embodiments; calcium stearoyl-2-lactylate (such as the calcium stearoyl-2-lactylate commercially available under the tradename VERV from American Ingredients Co., Kansas City, Mo.) may be utilized. Other fatty acid ester salts which may be utilized include lithium stearoyl lactylate, potassium stearoyl lactylate, rubidium stearoyl lactylate, cesium stearoyl lactylate, francium stearoyl lactylate, sodium palmityl lactylate, lithium palmityl lactylate, potassium palmityl lactylate, rubidium palmityl lactylate, cesium palmityl lactylate, francium palmityl lactylate, sodium olelyl lactylate, lithium olelyl lactylate, potassium olelyl lactylate, rubidium olelyl lactylate, cesium olelyl lactylate, and francium olelyl lactylate.

Where utilized, the amount of fatty acid component can be from about 5 percent to about 60 percent by weight of the total composition. In embodiments, the fatty acid component may be present in an amount from about 15 percent to about 55 percent by weight of the total composition.

In one embodiment, the hydroxamate-initiated polymer can be present in an amount from about 45 to about 60 weight percent of the composition and the fatty acid component, such as a fatty acid salt or a salt of a fatty acid ester, can be present in an amount from about 40 to about 55 weight percent of the composition. In embodiments, the hydroxamate-initiated polymer can be present in an amount from about 50 to about 55 weight percent of the composition and the fatty acid component can be present in an amount from about 45 to about 50 weight percent of the composition.

In other embodiments, the hydroxamate-initiated polymers of the present disclosure may be combined with additional polymeric materials, such as oligomers and/or polymers. The additional polymeric materials can be absorbable or non-absorbable. The additional polymeric materials may be blended with or bonded to (e.g., to create a block copolymer) the hydroxamate-initiated polymers of the present disclosure.

In embodiments, the hydroxamate-initiated polymers of the present disclosure may be combined with polyalkylene oxides such as polyethylene oxides, polyethylene glycol, polypropylene glycol, and the like. Such combinations may include blends or copolymers of the hydroxamate-initiated polymers of the present disclosure with the polyalkylene oxide oligomers or polymers. The resulting composition may thus possess MMP-inhibiting properties due to the presence of the hydroxamate-initiated polymers described above. Bioabsorbable polymers which may be utilized in the composition are within the purview of those skilled in the art and include those containing linkages derived from monomers including, for example, glycolide, lactide, glycolic acid, lactic acid, caprolactone, trimethylene carbonate, dioxanones, dioxepanones, and the like, and homopolymers, copolymers and combinations thereof.

Compositions including these hydroxamate-initiated polymers can also be used as coatings on textiles and medical devices noted above.

Textiles which may be made from or coated with compositions of the present disclosure include fibers made of hydroxamate-initiated polymers of the present disclosure, as well as other natural fibers, synthetic fibers, blends of natural fibers, blends of synthetic fibers, and blends of natural fibers with synthetic fibers. Suitable other materials utilized to form textiles include polyesters, polyamides, polyolefins, halogenated polymers, polyester/polyethers, polyurethanes, homopolymers thereof, copolymers thereof, and combinations thereof. Specific examples of suitable materials include polyethylene, polypropylene, polybutylene, polyvinyl chloride, polyethylene terephthalate, nylon 6, and nylon 6,6.

Medical devices may be formed of hydroxamate-initiated polymers of the present disclosure. In embodiments, medical devices can also be formed of absorbable materials, nonabsorbable materials, and combinations thereof. Suitable absorbable materials which may be utilized to form the medical device include trimethylene carbonate, caprolactone, dioxanone, glycolic acid, lactic acid, glycolide, lactide, homopolymers thereof, copolymers thereof, and combinations thereof. Suitable non-absorbable materials which may be utilized to form the medical device include polyolefins, such as polyethylene, polypropylene, copolymers of polyethylene and polypropylene, and blends of polyethylene and polypropylene.

As noted above, hydroxamate-initiated polymers of the present disclosure may also be used to form coatings for articles, including textiles and medical devices. In embodiments, the composition of the present disclosure can be applied as a solution and the solvent evaporated to leave the coating components, in embodiments, the hydroxamate-initiated polymer. Suitable solvents which may be utilized in forming the solution include any solvent or combination of solvents suitable for the chosen coating composition. To be suitable, the solvent should (1) be miscible with the coating components including the hydroxamate-initiated polymer, and (2) not appreciably affect the integrity of any material used to form the article being coated, such as a suture. Some examples of suitable solvents include alcohols, ketones, ethers, aldehydes, acetonitrile, acetic acid, methylene chloride, chloroform and water. In embodiments, methylene chloride may be used as a solvent.

Preparing a coating solution of the present disclosure is also a relatively simple procedure and can be accomplished by blending, mixing, and the like. In embodiments, where a hydroxamate-initiated polymer and methylene chloride are utilized to form the coating solution, the desired amount of hydroxamate-initiated polymer may be placed into a container, followed by the addition of the desired amount of methylene chloride. The two ingredients may then be mixed thoroughly to combine the ingredients. In embodiments, a fatty acid component as described above, including a calcium stearoyl lactate, may be included in the coating solution.

Any known technique may be employed for applying the coating, for example as a solution or suspension, to an article. Suitable techniques include dipping, spraying, wiping and brushing. The article wetted with the coating solution or suspension may be subsequently passed through or held in a drying oven for a time and at a temperature sufficient to vaporize and drive off the solvent.

In embodiments, a medical device in accordance with the present disclosure may be a suture. Sutures in accordance with the present disclosure may be monofilament or multifilament and may be made of any conventional material, including both bioabsorbable and non-bioabsorbable materials, such as surgical gut, silk, cotton, polyolefins such as polypropylene, polyamides, polyglycolic acids, polylactic acids, polyesters such as polyethylene terephthalate and glycolide-lactide copolymers, etc.

In embodiments, the suture may be made of a polyolefin. Suitable polyolefins include polyethylene, polypropylene, copolymers of polyethylene and polypropylene, and blends of polyethylene and polypropylene. In some embodiments, polypropylene can be utilized to form the suture. The polypropylene can be isotactic polypropylene or a mixture of isotactic and syndiotactic or atactic polypropylene.

In other embodiments, the suture may be made from synthetic absorbable polymers such as those made from glycolide, lactide, caprolactone, alkylene carbonates (i.e., trimethylene carbonate, tetramethylene carbonate, etc.), dioxanones, and copolymers and combinations thereof. One combination which may be utilized includes glycolide and lactide based polyesters, including copolymers of glycolide and lactide.

As noted above, the suture can be monofilament or multifilament. Where the suture is a monofilament, methods for producing such sutures are within the purview of those skilled in the art. Such methods include forming a suture material, such as a polyolefin resin, and extruding, drawing and annealing the resin to form the monofilament.

Where the sutures are made of multiple filaments, the suture can be made using any technique within the purview of one skilled in the art such as, for example, braiding, weaving or knitting. The filaments may also be combined to produce a non-woven suture. The filaments themselves may be drawn, oriented, crinkled, twisted, commingled or air entangled to form yarns as part of the suture forming process.

In embodiments a multifilament suture of the present disclosure can be produced by braiding. The braiding can be done by any method within the purview of those skilled in the art. For example, braid constructions for sutures and other medical devices are described in U.S. Patent Nos. 5,019,093; 5,059,213; 5,133,738; 5,181,923; 5,226,912; 5,261,886; 5,306,289; 5,318,575; 5,370,031; 5,383,387; 5,662,682; 5,667,528; and 6,203,564. Once the suture is constructed, it can be sterilized by any means within the purview of those skilled in the art.

In some cases a tubular braid, or sheath, can be constructed about a core structure which is fed through the center of a braider. Known tubular braided sutures, including those possessing cores, are disclosed, e.g., in U.S. Patent Nos. 3,187,752; 3,565,077; 4,014,973; 4,043,344; and 4,047,533.

In embodiments, a suture in accordance with the present disclosure may be attached to any surgical needle within the purview of those skilled in the art to produce a needled suture. Wounds may be sutured by passing a needled suture through tissue to create wound closure. The needle may then be removed from the suture and the suture tied. The suture may remain in the tissue and help promote wound healing by virtue of its MMP-inhibiting properties, thereby minimizing infection. The suture coating also advantageously enhances the surgeon's ability to pass the suture through tissue, and increases the ease and security with which he/she can tie the suture.

Medical devices in accordance with this disclosure can be sterilized in accordance with techniques within the purview of those skilled in the art.

If desired, in addition to the hydroxamate-initiated polymers of the present disclosure, compositions described herein can optionally contain additional components, e.g., dyes, antimicrobial agents, growth factors, anti-inflammatory agents, and the like. The term "antimicrobial agent" as used in the present disclosure includes antibiotics, antiseptics, disinfectants and combinations thereof.

Classes of antibiotics that can be combined with the hydroxamate-initiated polymers include tetracyclines like minocycline; rifamycins like rifampin; macrolides like erythromycin; penicillins like nafcillin; cephalosporins like cefazolin; beta-lactam antibiotics like imipenem and aztreonam; aminoglycosides like gentamicin and TOBRAMYCIN^{®}; chloramphenicol; sulfonamides like sulfamethoxazole; glycopeptides like vancomycin; quinolones like ciprofloxacin; fusidic acid; trimethoprim; metronidazole; clindamycin; mupirocin; polyenes like amphotericin B; azoles like fluconazole; beta-lactam inhibitors like sulbactam, and combinations of the foregoing.

Examples of antiseptics and disinfectants which may be combined with the hydroxamate-initiated polymers include hexachlorophene; cationic biguanides like chlorhexidine and cyclohexidine; iodine and iodophores like povidone-iodine; halo-substituted phenolic compounds like PCMX (i.e., p-chloro-m-xylenol) and triclosan (i.e., 2,4,4'-trichloro-2'hydroxy-diphenylether); furan medical preparations like nitrofurantoin and nitrofurazone; methenamine; aldehydes like glutaraldehyde and formaldehyde; alcohols, and combinations of the foregoing. In some embodiments, at least one of the antimicrobial agents may be an antiseptic, such as triclosan.

The hydroxamate-initiated polymers of the present disclosure may be combined with various optional ingredients, such as stabilizing agents, thickeners, colors, etc. The optional ingredients may represent up to about 10% of the total weight of the compositions formed with hydroxamate-initiated polymers of the present disclosure.

As low amounts of hydroxamates are required in compositions of the present disclosure, existing formulations and manufacturing processes need only minimal modifications to produce the compositions described herein. This ease of formulation and production may reduce both the time and cost necessary to prepare compositions of the present disclosure, compared with adding other MMP-inhibitors to existing materials.

While the above description contains many specifics, these specifics should not be construed as limitations on the scope of the disclosure herein but merely as exemplifications of particularly useful embodiments thereof. Those skilled in the art will envision many other possibilities within the scope of the disclosure as defined by the claims appended hereto.

## Claims

1. A hydroxamate-initiated polymer, wherein:
i) the hydroxamate initiator has the formula: 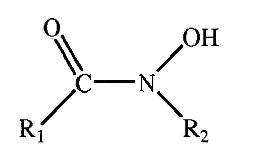 wherein R₁ is selected from the group consisting of vinyl groups, hydroxy acrylate groups, hydroxy methacrylate groups, acrylamide, methacrylamide, alkyl amines, alkyl groups, alkoxy groups, alkenyl groups, polymers terminated with the foregoing groups, and combinations thereof, and R₂ comprises hydrogen; and
ii) the hydroxamate-initiated polymer comprises a polymeric component, said polymeric component:
a) being formed from one or more lactones selected from the group consisting of lactide, glycolide, trimethylene carbonate, tetramethylene carbonate, dimethyl trimethylene carbonate, dioxanones, dioxepanones, caprolactone, valerolactone, and combinations thereof, optionally together with one or more monomer selected from absorbable cyclic amides, absorbable cyclic ether-esters derived from crown ethers, hydroxyl acids capable of esterification, polyalkyl ethers, and combinations thereof, or
b) being selected from polyesters, polyamides, polyester/polyethers, homopolymers thereof, copolymers thereof, and combinations thereof; and
wherein said hydroxamate is attached via a hydrolytically degradable bond at the head of the polymeric component.

2. The hydroxamate-initiated polymer of claim 1, wherein R₁ comprises a polymer functionalized with a group selected from the group consisting of vinyl alcohols, hydroxyethyl methacrylates, and combinations thereof.

3. The hydroxamate-initiated polymer of claim 1, wherein the polymeric component is formed from one or more of said lactone monomers copolymerized with at least one monomer selected from the group consisting of absorbable cyclic amides, absorbable cyclic ether-esters derived from crown ethers, alpha hydroxy acids, beta hydroxyacids, polyalkyl ethers, and combinations thereof.

4. The hydroxamate-initiated polymer of claim 1, wherein the polymeric component is formed from one or more of said lactone monomers copolymerized with at least one monomer selected from the group consisting of glycolic acid, lactic acid, beta hydroxybutyric acid, gamma hydroxyvaleric acid, polyethylene glycol, and combinations thereof.

5. An article comprising the hydroxamate-initiated polymer of claim 1, wherein the article comprises a medical device.

6. A coating comprising the hydroxamate-initiated polymer of claim 1.

7. A method comprising:
contacting one or more monomers with a hydroxamate-containing initiator under polymerizing conditions, the hydroxamate-containing initiator having the formula: 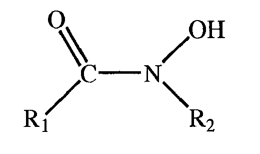 wherein R₁ is selected from the group consisting of vinyl groups, hydroxy acrylate groups, hydroxy methacrylate groups, acrylamide, methacrylamide, alkyl amines, alkyl groups, alkoxy groups, alkenyl groups, polymers terminated with the foregoing groups, and combinations thereof, and R₂ comprises hydrogen;
said one or more monomers being:
a) one or more lactone monomers selected from the group consisting of lactide, glycolide, trimethylene carbonate, tetramethylene carbonate, dimethyl trimethylene carbonate, dioxanones, dioxepanones, caprolactone, valerolactone, and combinations thereof, optionally together with one or more monomer selected from absorbable cyclic amides, absorbable cyclic ether-esters derived from crown ethers, hydroxyl acids capable of esterification, polyalkyl ethers, and combinations thereof, or
b) one or more monomers that form polyesters, polyamides, polyester/ polyethers, homopolymers thereof, copolymers thereof, and combinations thereof; and recovering a polymer comprising a polymeric component and the hydroxamate attached via a hydrolytically degradable bond at the head of the polymeric component.

8. The method of claim 7, wherein R₁ comprises a polymer functionalized with a component selected from the group consisting of vinyl alcohols, hydroxyethyl methacrylates, and combinations thereof; or the method of claim 7, wherein the one or more monomers are copolymers comprising one or more of said lactone monomers copolymerized with at least one monomer selected from the group consisting of absorbable cyclic amides, absorbable cyclic ether-esters derived from crown ethers, alpha hydroxy acids, beta hydroxyacids, polyalkyl ethers, and combinations thereof; or the method of claim 7, wherein the one or more monomers are copolymers comprising one or more of said lactone monomers copolymerized with at least one monomer selected from the group consisting of glycolic acid, lactic acid, beta hydroxybutyric acid, gamma hydroxyvaleric acid, polyethylene glycol, and combinations thereof.

9. The method of claim 7, wherein the one or more monomers and the hydroxamate initiator are heated at temperatures from 160° C to 200° C for a period of time from 4 hours to 30 hours.

10. A medical device comprising a hydroxamate-initiated polymer, wherein:
i) the hydroxamate initiator has the formula: 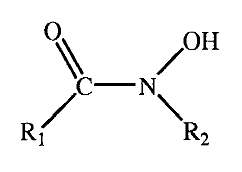 wherein R₁ is selected from the group consisting of vinyl groups, hydroxy acrylate groups, hydroxy methacrylate groups, acrylamide, methacrylamide, alkyl amines, alkyl groups, alkoxy groups, alkenyl groups, polymers terminated with the foregoing groups, and combinations thereof, and R₂ comprises hydrogen; and
ii) the hydroxamate-initiated polymer comprises a polymeric component, said polymeric component:
a) being formed from one or more lactones selected from the group consisting of lactide, glycolide, trimethylene carbonate, tetramethylene carbonate, dimethyl trimethylene carbonate, dioxanones, dioxepanones, caprolactone, valerolactone, and combinations thereof, optionally together with one or more monomer selected from absorbable cyclic amides, absorbable cyclic ether-esters derived from crown ethers, hydroxyl acids capable of esterification, polyalkyl ethers, and combinations thereof, or
b) being selected from polyesters, polyamides, polyester/polyethers, hompolymers thereof, copolymers thereof, and combinations thereof; and
wherein said hydroxamate is attached via a hydrolytically degradable bond at the head of the polymeric component.

11. The medical device of claim 10, wherein R₁ comprises a polymer functionalized with a component selected from the group consisting of vinyl alcohols, hydroxyethyl methacrylates, and combinations thereof.

12. The medical device of claim 10, wherein the polymeric component is formed from one or more of said lactone monomers copolymerized with at least one monomer selected from the group consisting of absorbable cyclic amides, absorbable cyclic ether-esters derived from crown ethers, alpha hydroxy acids, beta hydroxyacids, polyalkyl ethers, and combinations thereof.

13. The medical device of claim 10, wherein the medical device is selected from the group consisting of sutures, surgical meshes, contact lenses, intraocular lenses, staples, clips, buttresses, lapbands, catheters, bandages, stents, grafts, stent/grafts, knotless wound closures, sealants, adhesives, anti-adhesion devices, anchors, tunnels, bone fillers, synthetic tendons, synthetic ligaments, grafts, tissue scaffolds, pins, screws, orthopedic hardware, pacers, pacemakers, and implants.

14. The medical device of claim 10, wherein the polymeric component comprises one or more lactone monomers copolymerized with at least one monomer selected from the group consisting of glycolic acid, lactic acid, beta hydroxybutyric acid, gamma hydroxyvaleric acid, polyethylene glycol, and combinations thereof.

15. The medical device of claim 10, wherein at least a portion of a surface of the medical device possesses a coating comprising the hydroxamate-initiated polymer.

## Patentansprüche

1. Hydroxamat-initiiertes Polymer, bei welchem:
i) der Hydroxamat-Initiator die Formel 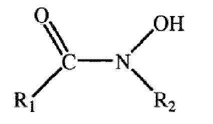 hat,
wobei R₁ ausgewählt ist aus der Gruppe bestehend aus Vinylgruppen, Hydroxyacrylatgruppen, Hydroxymethacrylatgruppen, Acrylamid, Methacrylamid, Alkylaminen, Alkylgruppen, Alkoxygruppen, Alkenylgruppen, mit den vorstehend genannten Gruppen beendeten Polymeren und Kombinationen davon, und R₂ Wasserstoff umfasst; und
ii) das Hydroxamat-initiierte Polymer eine Polymerkomponente aufweist, welche Polymerkomponente:
a) aus einem oder mehreren Lactonen gebildet ist, die ausgewählt sind aus der Gruppe bestehend aus Lactid, Glykolid, Trimethylencarbonat, Tetramethylencarbonat, Dimethyltrimethylencarbonat, Dioxanonen, Dioxepanonen, Caprolacton, Valerolacton und Kombinationen davon, optional zusammen mit einem oder mit mehreren Monomeren, die ausgewählt sind aus absorbierbaren zyklischen Amiden, absorbierbaren zyklischen Ether-Estern, die aus Kronenethern abgeleitet werden, zur Veresterung fähigen Hydroxylsäuren, Polyalkylethern und Kombinationen davon, oder
b) ausgewählt ist aus Polyestern, Polyamiden, Polyester/Polyethern, Homopolymeren davon, Copolymeren davon und Kombinationen davon; und
wobei das Hydroxamat durch eine hydrolytisch abbaubare Bindung am Kopf der Polymerkomponente befestigt ist.

2. Hydroxamat-initiiertes Polymer nach Anspruch 1, bei welchem R₁ ein Polymer umfasst, das mit einer Gruppe funktionalisiert ist, die ausgewählt ist aus der Gruppe bestehend aus Vinylalkoholen, Hydroxyethylmethacrylaten und Kombinationen davon.

3. Hydroxamat-initiiertes Polymer nach Anspruch 1, bei welchem die Polymerkomponente aus einem oder mehreren der Lactonmonomere gebildet ist, das mit mindestens einem Monomer copolymerisiert ist, das ausgewählt ist aus der Gruppe bestehend aus absorbierbaren zyklischen Amiden, absorbierbaren zyklischen Ether-Estern, die aus Kronenethern abgeleitet werden, alpha-Hydroxysäuren, beta-Hydroxysäuren, Polyalkylethern und Kombinationen davon.

4. Hydroxamat-initiiertes Polymer nach Anspruch 1, bei welchem die Polymerkomponente aus einem oder mehreren der Lactonmonomere gebildet ist, das mit mindestens einem Monomer copolymerisiert ist, das ausgewählt ist aus der Gruppe bestehend aus Glykolsäure, Milchsäure, beta-Hydroxybuttersäure, gamma-Valeriansäure, Polyethylenglykol und Kombinationen davon.

5. Gegenstand, enthaltend das Hydroxamat-initiierte Polymer nach Anspruch 1, wobei der Gegenstand eine medizinische Vorrichtung umfasst.

6. Beschichtung, enthaltend das Hydroxamat-initiierte Polymer nach Anspruch 1.

7. Verfahren, enthaltend:
Kontaktieren eines oder mehrerer Monomere mit einem Hydroxamat enthaltenden Initiator unter Polymerisationsbedingungen, wobei der Hydroxamat enthaltende Initiator die Formel hat: 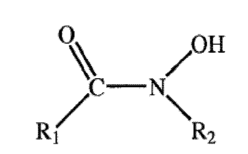
wobei R₁ ausgewählt ist aus der Gruppe bestehend aus Vinylgruppen, Hydroxyacrylatgruppen, Hydroxymethacrylatgruppen, Acrylamid, Methacrylamid, Alkylaminen, Alkylgruppen, Alkoxygruppen, Alkenylgruppen, mit den vorstehend genannten Gruppen beendeten Polymeren und Kombinationen davon, und R₂ Wasserstoff umfasst;
wobei das eine oder die Vielzahl der Monomere sind:
a) ein oder mehrere Lactonmonomere, die ausgewählt sind aus der Gruppe bestehend aus Lactid, Glykolid, Trimethylencarbonat, Tetramethylencarbonat, Dimethyltrimethylencarbonat, Dioxanonen, Dioxepanonen, Caprolacton, Valerolacton und Kombinationen davon, optional zusammen mit einem oder mit mehreren Monomeren, die ausgewählt sind aus absorbierbaren zyklischen Amiden, absorbierbaren zyklischen Ether-Estern, die aus Kronenethern abgeleitet werden, zur Veresterung fähigen Hydroxylsäuren, Polyalkylethern und Kombinationen davon, oder
b) ein oder mehreren Monomere, die Polyester, Polyamide, Polyester/Polyether, Homopolymere davon, Copolymere davon und Kombinationen davon bilden; und
Gewinnen eines Polymers, welches eine Polymerkomponente und das durch eine hydrolytisch abbaubare Bindung am Kopf der Polymerkomponente befestigte Hydroxamat enthält.

8. Verfahren nach Anspruch 7, bei welchem R₁ ein Polymer umfasst, das mit einer Komponente funktionalisiert ist, die ausgewählt ist aus der Gruppe bestehend aus Vinylalkoholen, Hydroxyethylmethacrylaten und Kombinationen davon; oder das Verfahren nach Anspruch 7, bei welchem das eine oder die Vielzahl von Monomeren Copolymere sind, die eines oder mehrere der Lactonmonomere enthalten, das mit mindestens einem Monomer copolymerisiert ist, das ausgewählt ist aus der Gruppe bestehend aus absorbierbaren zyklischen Amiden, absorbierbaren zyklischen Ether-Estern, die aus Kronenethern abgeleitet werden, alpha-Hydroxysäuren, beta-Hydroxysäuren, Polyalkylethern und Kombinationen davon; oder das Verfahren nach Anspruch 7, bei welchem das eine oder die Vielzahl von Monomeren Copolymere sind, die eines oder mehrere der Lactonmonomere enthalten, das mit mindestens einem Monomer copolymerisiert ist, das ausgewählt ist aus der Gruppe bestehend aus Glykolsäure, Milchsäure, beta-Hydroxybuttersäure, gamma-Valeriansäure, Polyethylenglykol und Kombinationen davon.

9. Verfahren nach Anspruch 7, bei welchem das eine oder die Vielzahl der Monomere und der Hydroxamat-Initiator für eine Zeitdauer von 4 h bis 30 h auf Temperaturen von 160 °C bis 200 °C erwärmt werden.

10. Medizinische Vorrichtung, enthaltend ein Hydroxamat-initiiertes Polymer, bei welcher:
i) der Hydroxamat-Initiator die Formel 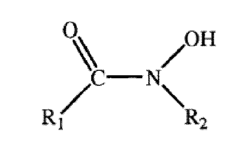 hat,
wobei R₁ ausgewählt ist aus der Gruppe bestehend aus Vinylgruppen, Hydroxyacrylatgruppen, Hydroxymethacrylatgruppen, Acrylamid, Methacrylamid, Alkylaminen, Alkylgruppen, Alkoxygruppen, Alkenylgruppen, mit den vorstehend genannten Gruppen beendeten Polymeren und Kombinationen davon, und R₂ Wasserstoff umfasst; und
ii) das Hydroxamat-initiierte Polymer eine Polymerkomponente aufweist, welche Polymerkomponente:
a) aus einem oder mehreren Lactonen gebildet ist, die ausgewählt sind aus der Gruppe bestehend aus Lactid, Glykolid, Trimethylencarbonat, Tetramethylencarbonat, Dimethyltrimethylencarbonat, Dioxanonen, Dioxepanonen, Caprolacton, Valerolacton und Kombinationen davon, optional zusammen mit einem oder mit mehreren Monomeren, die ausgewählt sind aus absorbierbaren zyklischen Amiden, absorbierbaren zyklischen Ether-Estern, die aus Kronenethern abgeleitet werden, zur Veresterung fähigen Hydroxylsäuren, Polyalkylethern und Kombinationen davon, oder
b) ausgewählt ist aus Polyestern, Polyamiden, Polyester/Polyethern, Homopolymeren davon, Copolymeren davon und Kombinationen davon; und
wobei das Hydroxamat durch eine hydrolytisch abbaubare Bindung am Kopf der Polymerkomponente befestigt ist.

11. Medizinische Vorrichtung nach Anspruch 10, bei welcher R₁ ein Polymer umfasst, das mit einer Gruppe funktionalisiert ist, die ausgewählt ist aus der Gruppe bestehend aus Vinylalkoholen, Hydroxyethylmethacrylaten und Kombinationen davon.

12. Medizinische Vorrichtung nach Anspruch 10, bei welcher die Polymerkomponente aus einem oder mehreren der Lactonmonomere gebildet ist, das mit mindestens einem Monomer copolymerisiert ist, das ausgewählt ist aus der Gruppe bestehend aus absorbierbaren zyklischen Amiden, absorbierbaren zyklischen Ether-Estern, die aus Kronenethern abgeleitet werden, alpha-Hydroxysäuren, beta-Hydroxysäuren, Polyalkylethern und Kombinationen davon.

13. Medizinische Vorrichtung nach Anspruch 10, bei welcher die medizinische Vorrichtung ausgewählt ist aus der Gruppe bestehend aus Nahtmaterialien, chirurgischen Netzen, Kontaktlinsen, Intraokularlinsen, Klammern, Clips, Verstärkungen, Magenbändern, Kathetern, Bandagen, Stents, Transplantaten, Stent/Transplantaten, knotenfreien Wundverschlüssen, Dichtungsmitteln, Klebstoffen, Antihaftmitteln, Ankern, Tunneln, Knochenfüllmaterialien, synthetischen Sehnen, synthetischen Bändern, Transplantaten, Gewebegerüsten, Stiften, Schrauben, orthopädischen Materialien, Schrittmachern, Herzschrittmachern und Implantaten.

14. Medizinische Vorrichtung nach Anspruch 10, bei welcher die Polymerkomponente ein oder mehrere Lactonmonomere umfasst, das mit mindestens einem Monomer copolymerisiert ist, das ausgewählt ist aus der Gruppe bestehend aus Glykolsäure, Milchsäure, beta-Hydroxybuttersäure, gamma-Valeriansäure, Polyethylenglykol und Kombinationen davon.

15. Medizinische Vorrichtung nach Anspruch 10, bei welcher zumindest ein Teil einer Oberfläche der medizinischen Vorrichtung eine Beschichtung besitzt, die ein Hydroxamat-initiiertes Polymer umfasst.

## Revendications

1. Polymère amorcé par un hydroxamate, dans lequel :
i) l'hydroxamate servant d'amorceur répond à la formule : 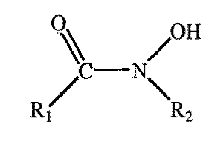 dans laquelle R₁ est sélectionné dans le groupe consistant en des groupements vinyliques, des groupements hydroxyacrylates, des groupements hydroxyméthacrylates, un acrylamide, un méthacrylamide, des alkylamines, des groupements alkyles, des groupements alkoxys, des groupements alcényles, des polymères terminés par les groupements susmentionnés et des combinaisons de ceux-ci et R₂ comprend un hydrogène ; et
ii) le polymère amorcé par un hydroxamate comprend un composant polymère, ledit composant polymère :
a) étant formé à partir d'une ou de plusieurs lactones sélectionnées dans le groupe consistant en un lactide, un glycolide, le carbonate de triméthylène, le carbonate de tétraméthylène, le carbonate de diméthyltriméthylène, des dioxanones, des dioxépanones, une caprolactone, une valérolactone et des combinaisons de ceux-ci, en option avec un ou plusieurs monomères sélectionnés parmi des amides cycliques absorbables, des éthers-esters cycliques absorbables dérivés d'éthers couronnes, des acides hydroxyles capables d'estérification, des éthers polyalkyliques et des combinaisons de ceux-ci ou
b) étant sélectionné parmi des polyesters, des polyamides, des polyesters/polyéthers, des homopolymères de ceux-ci, des copolymères de ceux-ci et des combinaisons de ceux-ci ; et
dans lequel ledit hydroxamate est rattaché à la tête du composant polymère par une liaison dégradable par hydrolyse.

2. Polymère amorcé par un hydroxamate de la revendication 1, dans lequel R₁ comprend un polymère fonctionnalisé par un groupement sélectionné dans le groupe consistant en des alcools vinyliques, des méthacrylates d'hydroxyéthyle et des combinaisons de ceux-ci.

3. Polymère amorcé par un hydroxamate de la revendication 1, dans lequel le composant polymère est formé à partir d'un ou de plusieurs desdits monomères lactones copolymérisés avec au moins un monomère sélectionné dans le groupe consistant en des amides cycliques absorbables, des éthers-esters cycliques absorbables dérivés d'éthers couronnes, des alpha-hydroxy acides, des bêta-hydroxy acides, des éthers polyalkyliques et des combinaisons de ceux-ci.

4. Polymère amorcé par un hydroxamate de la revendication 1, dans lequel le composant polymère est formé à partir d'un ou de plusieurs desdits monomères lactones copolymérisés avec au moins un monomère sélectionné dans le groupe consistant en l'acide glycolique, l'acide lactique, l'acide bêta-hydroxybutyrique, l'acide gamma-hydroxyvalérique, le polyéthylène-glycol et des combinaisons de ceux-ci.

5. Article comprenant le polymère amorcé par un hydroxamate de la revendication 1, ledit article comprenant un dispositif médical.

6. Revêtement comprenant le polymère amorcé par un hydroxamate de la revendication 1.

7. Méthode comprenant :
la mise en contact d'un ou de plusieurs monomères avec un amorceur contenant un hydroxamate dans des conditions de polymérisation, l'amorceur contenant un hydroxamate répondant à la formule : 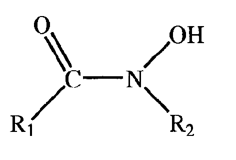 dans laquelle R₁ est sélectionné dans le groupe consistant en des groupements vinyliques, des groupements hydroxyacrylates, des groupements hydroxyméthacrylates, un acrylamide, un méthacrylamide, des alkylamines, des groupements alkyles, des groupements alkoxys, des groupements alcényles, des polymères terminés par les groupements susmentionnés et des combinaisons de ceux-ci et R₂ comprend un hydrogène ;
lesdits un ou plusieurs monomères étant :
a) un ou plusieurs monomères lactones sélectionnés dans le groupe consistant en un lactide, un glycolide, le carbonate de triméthylène, le carbonate de tétraméthylène, le carbonate de diméthyltriméthylène, des dioxanones, des dioxépanones, une caprolactone, une valérolactone et des combinaisons de ceux-ci, en option avec un ou plusieurs monomères sélectionnés parmi des amides cycliques absorbables, des éthers-esters cycliques absorbables dérivés d'éthers couronnes, des acides hydroxyles capables d'estérification, des éthers polyalkyliques et des combinaisons de ceux-ci ou
b) un ou plusieurs monomères qui forment des polyesters, des polyamides, des polyesters/polyéthers, des homopolymères de ceux-ci, des copolymères de ceux-ci et des combinaisons de ceux-ci ; et
la récupération d'un polymère comprenant un composant polymère et l'hydroxamate rattaché à la tête du composant polymère par une liaison dégradable par hydrolyse.

8. Méthode de la revendication 7, dans laquelle R₁ comprend un polymère fonctionnalisé par un composant sélectionné dans le groupe consistant en des alcools vinyliques, des méthacrylates d'hydroxyéthyle et des combinaisons de ceux-ci ; ou méthode de la revendication 7 dans laquelle les un ou plusieurs monomères sont des copolymères comprenant un ou plusieurs desdits monomères lactones copolymérisés avec au moins un monomère sélectionné dans le groupe consistant en des amides cycliques absorbables, des éthers-esters cycliques absorbables dérivés d'éthers couronnes, des alpha-hydroxy acides, des bêta-hydroxy acides, des éthers polyalkyliques et des combinaisons de ceux-ci ; ou méthode de la revendication 7 dans laquelle les uns ou plusieurs monomères sont des copolymères comprenant un ou plusieurs desdits monomères lactones copolymérisés avec au moins un monomère sélectionné dans le groupe consistant en l'acide glycolique, l'acide lactique, l'acide bêta-hydroxybutyrique, l'acide gamma-hydroxyvalérique, le polyéthylène-glycol et des combinaisons de ceux-ci.

9. Méthode de la revendication 7, dans laquelle les un ou plusieurs monomères et l'hydroxamate servant d'amorceur sont chauffés à des températures allant de 160 °C à 200 °C pendant une période d'une durée de 4 heures à 30 heures.

10. Dispositif médical comprenant un polymère amorcé par un hydroxamate, dans lequel :
i) l'hydroxamate servant d'amorceur répond à la formule : 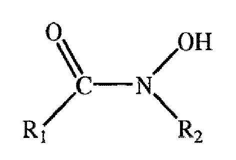 dans laquelle R₁ est sélectionné dans le groupe consistant en des groupements vinyliques, des groupements hydroxyacrylates, des groupements hydroxyméthacrylates, un acrylamide, un méthacrylamide, des alkylamines, des groupements alkyles, des groupements alkoxys, des groupements alcényles, des polymères terminés par les groupements susmentionnés et des combinaisons de ceux-ci et R₂ comprend un hydrogène ; et
ii) le polymère amorcé par un hydroxamate comprend un composant polymère, ledit composant polymère :
a) étant formé à partir d'une ou de plusieurs lactones sélectionnées dans le groupe consistant en un lactide, un glycolide, le carbonate de triméthylène, le carbonate de tétraméthylène, le carbonate de diméthyltriméthylène, des dioxanones, des dioxépanones, une caprolactone, une valérolactone et des combinaisons de ceux-ci, en option avec un ou plusieurs monomères sélectionnés parmi des amides cycliques absorbables, des éthers-esters cycliques absorbables dérivés d'éthers couronnes, des acides hydroxyles capables d'estérification, des éthers polyalkyliques et des combinaisons de ceux-ci ou
b) étant sélectionné parmi des polyesters, des polyamides, des polyesters/polyéthers, des homopolymères de ceux-ci, des copolymères de ceux-ci et des combinaisons de ceux-ci ; et
dans lequel ledit hydroxamate est rattaché à la tête du composant polymère par une liaison dégradable par hydrolyse.

11. Dispositif médical de la revendication 10, dans lequel R₁ comprend un polymère fonctionnalisé par un composant sélectionné dans le groupe consistant en des alcools vinyliques, des méthacrylates d'hydroxyéthyle et des combinaisons de ceux-ci.

12. Dispositif médical de la revendication 10, dans lequel le composant polymère est formé à partir d'un ou de plusieurs desdits monomères lactones copolymérisés avec au moins un monomère sélectionné dans le groupe consistant en des amides cycliques absorbables, des éthers-esters cycliques absorbables dérivés d'éthers couronnes, des alpha-hydroxy acides, des bêta-hydroxy acides, des éthers polyalkyliques et des combinaisons de ceux-ci.

13. Dispositif médical de la revendication 10, le dispositif médical étant sélectionné dans le groupe consistant en les suivants : sutures, filets chirurgicaux, lentilles de contact, lentilles intraoculaires, agrafes, clips, pièces d'appui, anneaux gastriques, cathéters, bandages, stents, prothèses, prothèses endovasculaires, fils de suture sans noeud, obturateurs, adhésifs, dispositifs anti-adhérentiels, systèmes d'ancrage, tunnels, matériaux de comblement osseux, tendons synthétiques, ligaments synthétiques, greffons, échafaudages tissulaires, broches, vis, matériel orthopédique, dispositifs d'entraînement, stimulateurs cardiaques et implants.

14. Dispositif médical de la revendication 10, dans lequel le composant polymère comprend un ou plusieurs monomères lactones copolymérisés avec au moins un monomère sélectionné dans le groupe consistant en l'acide glycolique, l'acide lactique, l'acide bêta-hydroxybutyrique, l'acide gamma-hydroxyvalérique, le polyéthylène-glycol et des combinaisons de ceux-ci.

15. Dispositif médical de la revendication 10, dans lequel au moins une portion d'une surface du dispositif médical présente un revêtement comprenant le polymère amorcé par un hydroxamate.
